# EUROPEAN PATENT SPECIFICATION

(11) **EP 2 181 693 B1**
(45) Date of publication and mention of the grant of the patent: **14.02.2018**
(21) Application number: 10151632.6
(22) Date of filing: 13.08.2004
(51) Int. Cl.: A61K 8/86, A61K 8/22, A61K 8/81, A61K 8/25, A61K 8/90, A61Q 11/00

(54) **Non-aqueous liquid tooth whitening composition**
Nicht-wässriges flüssiges Zahnbleichmittel
Composition liquide non-aqueuse pour le blanchiment des dents

(30) Priority: 15.08.2003 US 641961
(43) Date of publication of application: 05.05.2010
(62) Divisional of application: 04780974.4
(73) Proprietor: Colgate-Palmolive Company, New York, NY 10022 (US)
(72) Inventor: Fei, Lin, Kendall Park, NJ 08824 (US); Chopra, Suman, K., Monroe, NJ 08831 (US); Mandadi, Prakasarao, Flemington, NJ 08822 (US); Patel, Neeta, Monmouth Junction, NJ 08852 (US); Shastry, Ramachandra, Dayton, NJ 08810 (US); Mirajkar, Yelloji-Rao, K., Piscataway, NJ 08854 (US); Prencipe, Michael, West Windsor, NJ 08550 (US)
(74) Representative: Wibbelmann, Jobst

(56) References cited:
- WO-A-00/19971
- WO-A-91/07184
- WO-A-97/11676
- WO-A1-03/000216
- US-A- 3 951 838
- US-A- 4 971 782
- US-A- 5 374 368
- US-A1- 2001 021 374
- US-B1- 6 306 370

## Description

### BACKGROUND OF THE INVENTION

### 1. Field of the Invention

This invention relates to stable nonaqueous liquid tooth whitening composition.

### 2. The Prior Art

It has become desirable for a person's teeth to appear bright or "white". Society places a high value on the "whiteness" of one's teeth. One whose teeth are white may enjoy more personal confidence and satisfaction and may even enjoy greater social acceptance.

A tooth is comprised of an inner dentin layer and an outer hard enamel layer that is the protective layer of the tooth. The enamel layer of a tooth is naturally an opaque white or slightly off-white color. It is the enamel layer that can become stained or discolored. The enamel layer of a tooth is composed of hydroxyapatite mineral crystals that create a somewhat porous surface. These hydroxyapatite crystals form microscopic hexagonal rods or prisms that make up the enamel surface. As a result, the surface of the enamel layer presents microscopic spaces or pores between the prisms. It is believed that this porous nature of the enamel layer is what allows staining agents and discoloring substances to permeate the enamel and discolor the tooth. These remaining substances can occupy the microscopic spaces and eventually alter the color of the tooth.

Many substances that a person confronts or comes in contact with on a daily basis can "stain" or reduce the "whiteness" of one's teeth. In particular, the foods, tobacco products and fluids that one consumes tend to stain one's teeth. These products or substances tend to accumulate on the enamel layer of the tooth and form a pellicle film over the teeth. These staining and discoloring substances can then permeate the enamel layer. This problem occurs gradually over many years, but imparts a noticeable discoloration of the enamel of one's teeth. So long as the discolored teeth are still healthy and do not pose any health risk or problem, a product or substance that would whiten the discolored teeth would be advantageous.

It is also essential that a tooth whitening product that is to be used at home or in private by the consumer be safe and easy to use and be stable and retain its whitening efficacy during its storage on retail store shelves as well as over the period of use by the consumer.

Products and substances that are presently available to whiten teeth include a variety of different ingredients, but the primary active ingredient is a peroxide agent formulated into an aqueous liquid, solution, paste or gel. These products upon storage lose their whitening efficacy over time as peroxide compounds in aqueous solutions are relatively unstable. This tendency toward instability of peroxide has limited the utility of aqueous liquid whitening products for whitening teeth. It would be highly desirable, therefore, to provide a stable peroxide whitening liquid to effect substantive whitening. US-A-2001/0021374 discloses tooth whitening compositions, including a low molecular weight component having a high acetyl group functionality useful in the production of a peroxy acid which acts as a whitening agent.

### SUMMARY OF THE INVENTION

The present invention provides a stable, non-aqueous liquid tooth whitening composition according to claim 1 and a method for whitening teeth according to claim 6 Preferred features are defined in the dependent claims.

In accordance with one aspect of this invention there is provided a stable non-aqueous dental whitening composition comprised of an anhydrous liquid suspension of an anhydrous peroxide whitening agent in an anhydrous liquid hydrophilic polymer, the suspension when applied to the teeth being sufficiently viscous to form an adherent, continuous layer of the peroxide containing composition on dental enamel surfaces.

The anhydrous liquid hydrophilic polymer of the present invention provides a stable vehicle that prevents the decomposition of the peroxide whitening agent during storage and before use. Once applied on tooth surface, the saliva on the tooth enamel surface to which the composition is applied will either dissolve or disintegrate the peroxide containing layer resulting in a rapid decomposition of the peroxide, which in turn provides the whitening effect.

The non-aqueous liquid whitening composition of the present invention is a portable oral care tooth whitener that can be conveniently painted onto the tooth enamel surface. Upon the paint-on application to the teeth, the applied nonaqueous liquid whitening composition forms an adherent layer of peroxide containing suspension that has the capacity to release the peroxide whitening agent over an extended period of time, e.g., from about 5 to about 30 minutes. The applied layer adheres to the tooth surface whereby the released peroxide source then whitens the teeth to which the composition is applied. The maintenance of the composition in a non-aqueous state provides a peroxide source that is substantially stable to decomposition on storage.

### DESCRIPTION OF THE PREFERRED EMBODIMENTS

The term "hydrophilic" polymer as employed herein refers to an organic polymer which has a water solubility of at least about one gram per 100 grams of water at 25°C. The term "hydrophobic" polymer or "water-insoluble" polymer as employed herein refers to an organic polymer which has a water solubility of less than about one gram per 100 grams of water at 25°C. The term "non-aqueous" as used herein means the presence of less than 10% by weight water in the composition of the present invention.

The composition of the present invention is a viscous suspension which maintains its consistency during storage enabling the product to be painted on the tooth surface with a soft applicator brush or applied from a coating layer on a strip.

### Non-Aqueous Hydrophilic Liquid Polymer

In accordance with the invention the anhydrous hydrophilic liquid polymer employed as the vehicle for the peroxide is polyethylene glycol Nonaqueous hydrophilic polymers useful in the practice of the present invention preferably provide a viscosity for the composition in the range between about 10,000 cps to 600,000 cps.

The compositions of the present invention may include as polyethylene glycols, nonionic polymers of ethylene oxide having the general formula:

HOCH₂ (CH₂OCH₂)ₙOH

wherein n represents the average number of oxyethylene groups. Polyethylene glycols available from Dow Chemical are designated by a number such as 200, 300, 400, 600, 2000 which represents the approximate average molecular weight of the polymer. Polyethylene glycols 200, 300, 400 and 600 are clear viscous liquids at room temperature, and are preferred for use in the practice of the present invention.

The polyethylene glycol vehicle employed in the compositions of the invention can be present in an amount of from about 5 to 85% by weight. Preferably, the liquid polymer is present in an amount of from about 55 to about 70% by weight.

### Adhesion Enhancing Agents

Adhesion enhancing agents are used in the compositions of the present invention to enhance the adhesive properties of the anhydrous hydrophilic polymers The adhesion enhancing agent is a polyvinyl pyrrolidone/vinyl acetate copolymer Small amounts of an anhydrous solvent such as ethanol as well as humectants such as glycerin may also be used to adjust the viscosity of the liquid compositions of the present invention and are present in the nonaqueous liquid whitening compositions of the present invention in amounts of about 0.1 to about 25% by weight and preferably about 0.3 to about 20% by weight.

### Anhydrous Whitening Agent

The anhydrous whitening agent useful in the practice of the present invention is a PVP-H₂0₂ complex (hereinafter "PVP-H₂0₂"). PVP-H₂0₂ both linear and cross linked complexes are known to the art and are disclosed in US 3,376,110 and US 3,480,557 and have been used in compositions for treating acne vulgaris (US5,122,370). PVP-H₂0₂ complexes are disclosed in US 5,122,370. PVP-H₂0₂ is stable in an anhydrous environment. By exposure to aqueous environments, as in the oral cavity, the whitening agent dissociates into individual species (PVP polymer and H₂0₂). The PVP-H₂0₂ complex is generally comprised of about 80% by weight polyvinyl pyrrolidone and 20% by weight H₂0₂.

The PVP/H₂O₂ complex can be present in the liquid whitening compositions of the present invention at a concentration of about 1 to about 50% by weight and preferably about 10 to about 25% by weight.

### Surfactant

Nonionic surfactants which are compatible with peroxide compounds serve as a solubilizing, dispersing, emulsifing and wetting agents and are especially effective to solubilize a flavor if included in the liquid whitening composition. A particularly useful nonionic surfactant is a water soluble polyoxyethylene monoester of sorbitol with a C10 to C18 fatty acid, marketed commercial under the Tween trademark. The Tween surfactants are mixtures of C10 to C18 fatty acid esters of sorbitol (and sorbitol anhydrides), consisting predominately of the monoester, condensed with about 10-30, preferably about 20, moles of ethyleneoxide. The fatty acid (aliphatic hydrocarbonyl monocarboxylic acid) may be saturated or unsaturated, e.g., lauric, palmitic, stearic, oleic acids. Polysorbate 20 (e.g., Tween 20) is especially preferred and is commonly referred to as polyoxyethylene (20) sorbitan monolaurate. The nonionic surfactant constitutes about 0 to 50% by weight and preferably 0.5 to 40% by weight of the liquid composition.

### Flavor

The nonaqueous liquid whitening composition of the present invention may also contain a flavoring agent. Flavoring agents that are used in the practice of the present invention include essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent is incorporated in the whitening liquid composition of the present invention at a concentration of about 0.1 to about 2% by weight and preferably about 0.1 to about 0.5% by weight.

A sweetening material is also employed as a complement to the flavoring material. Suitable sweetening agents are water soluble and include sodium saccharin, sodium cyclamate, xylitol, perillartien, D-tryptophan, aspartame, dihydrochalcones and the like, in concentrations of about 0.01 to about 1% by weight. Sodium saccharin is preferred.

The nonaqueous liquid whitening composition of the present invention is prepared in the form of a flowable viscous liquid suspension containing the whitening agent and is applied as such to the users teeth as by painting the teeth with a soft applicator brush. Application by the user, leaves a coating of the thick liquid suspension on the teeth. Contact with saliva causes the slow release of H₂0₂ to the applied tooth site from the anhydrous whitening compound providing prolonged whitening treatment of the tooth sites.

The layer of liquid peroxide containing suspension contains no ingredients imparting thereto an unacceptable taste or texture, rendering it unpleasant to the user and adheres strongly to tooth enamel. The suspension is sufficiently viscous and adherent enough to remain on the teeth for a period of time, for example about 5 to about 30 minutes to effect a whitening result and will resist the forces commonly applied by the lips and tongue. While the layer of applied liquid whitening composition is in place, the user is to refrain from mastication. The whitening composition can be removed as and when required, at will, by an employment of standard oral hygiene procedures such as brushing or by rinsing with an alcoholic mouthwash. While in place the coating releases agents contained therein at a slow, relatively constant rate and in concentration sufficient effectively to effect stain removal from the teeth.

### Peroxide Activators

Peroxide decomposition activators such as sodium bicarbonate, sodium carbonate, manganese gluconate may be incorporated in the liquid whitening gel composition of the present invention. The activator is relatively nonactive with the peroxide whitening agent when present in the stored nonaqueous liquid composition due to the anhydrous compositions. The activator functions to react with the peroxide to release oxygen when the liquid whitening composition applied to the teeth is contacted with saliva in the oral cavity. The peroxide activator is present in the non-aqueous liquid composition of the present invention at a concentration of about 0 to about 10 % by weight and preferably about 1 to about 5 % by weight.

Other ingredients which may be incorporated in the liquid whitening composition of the present invention include any of the materials commonly used in the oral care formulations. These include: antimicrobial agents, e.g., Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol); antiinflammatory agents such as ibuprofen, flurbiprofen, aspirin, indomethacine etc.; anticaries agents such as sodium-, calcium-, magnesium- and stannous fluoride, aminefluorides, disodium monofluorophosphate and sodium trimeta phosphate; plaque buffers such as urea, calcium lactate, calcium glycerophosphate and strontium polyacrylates; vitamins such as Vitamin C; plant extracts; desensitizing agents, e.g., potassium citrate, potassium chloride, potassium tartrate, potassium bicarbonate, potassium oxalate, potassium nitrate and strontium salts; agents effective against dental calculus such as pyrophosphate salts including the mono, di, tri and tetra alkali metal and ammonium pyrophosphate and tripolyphosphate salts; biomolecules, e.g., bacteriocins, antibodies, enzymes such as papain, glucoamylase; opacifying agents, pigments, coloring agents and fluoride ion providing salts having anticaries efficacy such as sodium fluoride, potassium fluoride, a tin fluoride such as stannous fluoride.

### Composition Preparation

The nonaqueous liquid whitening compositions of the present invention are prepared by adding and mixing the ingredients of the composition in a suitable vessel such as a stainless steel tank provided with a mixer. In the preparation of the liquid whitening composition, the ingredients are advantageously added to the mixer in the following order: hydrophilic polymer, anhydrous peroxide compound whitening agent, adhesive enhancer, peroxide activation and any desired flavoring or sweetener. The ingredients are then mixed to form a homogeneous dispersion/solution.

The present invention is illustrated by the following examples but is not to be limited thereby.

### Comparative examples

A series of liquid whitening paint-on compositions not according to the invention were prepared using the ingredients listed in Table I below.

| **TABLE I** | | | | | | | |
|---|---|---|---|---|---|---|---|
| | **Wt. %** | | | | | | |
| **Composition** | **A** | **B** | **C** | **D** | **E** | **F** | **G** |
| **Ingredient** | | | | | | | |
| PEG 400 | 71.20 | 33.50 | 51.50 | 21.50 | 12.50 | 37.00 | -- |
| Polyethylene/Polypropylene block copolymer* | -- | --- | -- | 50.00 | 60.00 | 35.00 | 54.5 |
| Amorphous silica** | 3.00 | 1.50 | 3.00 | 1.50 | 0.50 | 3.00 | -- |
| Sodium percarbonate | 24.00 | 24.00 | 24.00 | 25.00 | 25.00 | 24.00 | 24.00 |
| Vinyl acetate copolymer | 0.50 | -- | -- | -- | -- | -- | -- |
| Anhydrous ethyl alcohol | 0.50 | -- | -- | 0.50 | 0.50 | -- | -- |
| Polyoxyethylene sorbitan*** | -- | 40.00 | -- | -- | -- | -- | -- |
| Dimethicone (350 cst) | -- | -- | -- | -- | -- | -- | 20.00 |
| PVPNA copolymer | -- | -- | 20.00 | -- | -- | -- | -- |
| Petrolatum | -- | -- | -- | -- | -- | -- | -- |
| Na saccharin | 0.80 | 1.00 | 1.00 | 1.00 | 1.00 | 1.00 | 1.00 |
| Flavor | -- | -- | 0.50 | 0.50 | 0.50 | -- | 0.5 |

| | | | | | | | |
|---|---|---|---|---|---|---|---|
| * Pluraflo L-1220 ** Aerosil 200 *** Polysorbate 60 | | | | | | | |

### Stability

The shelf stability of the nonaqueous liquid whitening composition of Table I was determined by packaging the composition in sealed plastic bottles and exposing the bottles to 49°C (120°F). The percent hydrogen peroxide recovered from the liquid whitening composition after a 4 week exposure to temperatures of 49°C (120 °F) determined using Iodometric Titration. The peroxide recovery results indicated that the nonaqueous liquid whitening compositions to be efficacious whitening compositions even after 4 weeks of storage at the elevated temperature of 120°F. The whitening efficacy of the liquid compositions was determined using a duplicate pair of flow cells designed to accommodate a total of eight bovine enamel blocks (four in each cell). The bovine enamel blocks were obtained freshly stained using an established staining protocol (Indiana University, Indianapolis, IN). The initial L*, a* and b* values were matched as closely as possible prior to the experiment using a chromameter (Minolta CR-321) based on initial L*, a* and b* values (CIELAB). These initial values were typically L* = 25.00, a* = 3.00, and b* = 5.00 to L* = 35.00, a* = 5.00, and b* = 7.00. The L, a, b values were measured four times at slightly differing locations on the surface of the bovine enamel blocks.

To simulate the saliva of the human mouth, an artificial saliva buffer solution maintained at 37°C was prepared which contained the salts usually present in saliva at levels typical to the levels found in human saliva.

The bovine enamel blocks were placed in the flow cells and the liquid compositions evenly applied using a brush, the amount of product applied being determined using the weight difference of the container. Flow over the teeth was 0.6 ml/min. for 30 min. Average initial and final chromometer readings were used to calculate ΔE according to ΔE = ((L_{f}-Lᵢ)² + (b_{f}-bᵢ)² + (a_{f}-aᵢ)²)^{½}. The final ΔE reported was the average over all observations after the rejection of outliers using the Student's test (95% confidence level). The results are recorded in Table II below. For purposes of comparison, the whitening efficacy test procedure of the comparative example was repeated with the exception that a commercially available aqueous paint-on tooth whitening composition designated composition X containing 6.75% by weight hydrogen peroxide was also evaluated for whitening efficacy. The results of these evaluation tests are recorded in Table II below.

| **TABLE II** | |
|---|---|
| **Whitening Efficacy** | |
| **Liquid Gel Formula** | **ΔE** |
| A | 17 |
| B | 17 |
| C | 17 |
| D | 34 |
| E | 20 |
| F | 26 |
| G | 26 |
| X | 5.3 |
| Y | 5.3 |

The results recorded in Table II indicate that the whitening efficacy (ΔE) of the (Compositions A-G) are substantially more efficacious than the comparative commercially available liquid tooth whitening compositions X and Y.

### Example

A series of anhydrous liquid whitening gel compositions designated "Compositions I-O" were prepared with either PVP-H₂0₂ or sodium percarbonate or combinations of the two anhydrous peroxide compounds were incorporated in a nonaqueous liquid vehicle in which a polyvinyl pyrolidone/vinyl acetate copolymer (PVP/VA) is also present. The ingredients of these compositions are listed in Table III below. (compositions N and O are not part of the invention)

| **TABLE III** | | | | | | | |
|---|---|---|---|---|---|---|---|
| **Composition** | **I (Wt.%)** | **J (Wt.%)** | **K (Wt.%)** | **L (Wt.%)** | **M (Wt.%)** | **N (Wt.%)** | **O (Wt.%)** |
| **Ingredients** | | | | | | | |
| Na percarbonate | -- | -- | -- | -- | 2 | 24 | 24 |
| PVP-hydrogen peroxide | 30 | 30 | 20 | 10 | 30 | -- | -- |
| PEG 600 | 62.9 | -- | 73 | 85 | 64 | 70.9 | 72 |
| PEG 400 | -- | 62.5 | -- | -- | -- | -- | -- |
| Fumed silica | 5 | 5 | 5 | 3 | 3 | 5 | 3 |
| NaHC0₃ | 2 | 2 | 1 | -- | -- | -- | -- |
| NaCO₃ | -- | -- | -- | 1 | -- | -- | -- |
| PVP/VA polymer | 0.1 | 0.5 | 1 | 1 | 1 | 0.1 | 1 |
| **Total** | **100.00** | **100.00** | **100.00** | **100.00** | **100.00** | **100.00** | **100.00** |

### Preparation of Compositions I-M

The polyethylene glycols (PEG) were added to PVPH₂0₂ with gentle mixing to form a slurry. The mixture was then heated to about 70°C with continuous stirring until the PVP H₂0₂ was fully dissolved in the PEG mixture. Then PVP/VA polymer is added to the PEG vehicle followed by the addition of fumed silica. The mixture was homogenized until a homogenous mixture was formed (about 10 minutes). Solid activator (NaHCO₃) was added to the mixture at the last stage, and stirred uniformly, dispense the activator.

### Preparation of Compositions N-O

PVP/VA polymer was dissolved in the PEG vehicle with stirring. Fumed silica was added followed by mixing until the mixture becomes visually uniform. Sodium percarbonate was then added to the mixture and stirred to prepare a homogeneous composition.

The whitening efficacy of the liquid whitening gel formulations I and J was determined using bovine teeth following the procedure of Example I.

| **TABLE IV** | |
|---|---|
| **Whitening Efficacy** | |
| **Liquid Gel Formula** | **ΔE** |
| I | 20 |
| J | 20 |

## Claims

1. A stable, non-aqueous liquid tooth whitening composition suitable for application to teeth in the oral cavity comprising an anhydrous peroxide compound dispersed in an orally acceptable anhydrous hydrophilic liquid polymer and a adhesion enhancing agent, wherein the composition is homogeneous, the anhydrous peroxide compound is PVP/H₂O₂, the anhydrous hydrophilic polymer is polyethylene glycol and the adhesion enhancing agent is a polyvinyl pyrrolidone/vinyl acetate copolymer, which composition when applied topically to tooth surfaces forms an adherent layer containing the peroxide compound which peroxide compound is thereafter released from the layer to effect tooth whitening.

2. The composition of claim 1 wherein the adhesion enhancing agent is present in the composition in an amount from 0.1 to 4 weight %.

3. The composition of claim 1 wherein a peroxide activator is present in the composition.

4. The composition of claim 3 wherein the peroxide activator is NaHCO₃, Na₂CO₃ or manganese gluconate.

5. The composition of claim 3 wherein the peroxide activator is present in the composition at a concentration of 1% to 10% by weight.

6. A method for whitening teeth which comprises
a) preparing a nonaqueous liquid tooth whitening composition according to claim 1;
b) painting the composition into contact with the teeth to be whitened;
c) maintaining the composition in contact with the teeth for plurality of minutes per day; and then,
d) repeating steps b and c for multiple days to thereby whiten the teeth.

## Patentansprüche

1. Stabile, nicht wässrige flüssige Zahnbleichmittelzusammensetzung, die zur Anwendung auf Zähne in der Mundhöhle geeignet ist, die eine wasserfreie Peroxidverbindung umfasst, die in einem oral verträglichen wasserfreien hydrophilen flüssigen Polymer und einem adhäsionsverbessernden Mittel dispergiert ist, wobei die Zusammensetzung homogen ist, die wasserfreie Peroxidverbindung PVP/H₂O₂ ist, das wasserfreie hydrophile Polymer Polyethylenglycol ist, und das adhäsionsverbessernde Mittel ein Polyvinylpyrrolidon/Vinylacetat-Copolymer ist, welche Zusammensetzung, wenn sie topisch auf Zahnoberflächen aufgetragen wird, eine adhärente Schicht bildet, die die Peroxidverbindung enthält, welche Peroxidverbindung danach aus der Schicht freigesetzt wird, um Zahnbleichen zu bewirken.

2. Zusammensetzung nach Anspruch 1, wobei das adhäsionsverbessernde Mittel in der Zusammensetzung in einer Menge von 0,1 bis 4 Gew.-% vorliegt.

3. Zusammensetzung nach Anspruch 1, wobei ein Peroxidaktivator in der Zusammensetzung vorliegt.

4. Zusammensetzung nach Anspruch 3, wobei der Peroxidaktivator NaHCO₃, Na₂CO₃ oder Mangangluconat ist.

5. Zusammensetzung nach Anspruch 3, wobei der Peroxidaktivator in der Zusammensetzung in einer Konzentration von 1% bis 10% bezogen auf das Gewicht vorliegt.

6. Verfahren zum Bleichen von Zähnen, das umfasst
a) Herstellen einer nicht wässrigen flüssigen Zahnbleichmittelzusammensetzung gemäß Anspruch 1;
b) Aufmalen der Zusammensetzung in Kontakt mit den zu bleichenden Zähnen;
c) Halten der Zusammensetzung in Kontakt mit den Zähnen für eine Vielzahl an Minuten pro Tag; und dann
d) Wiederholen von Schritten b und c für mehrere Tage, um dadurch die Zähne zu bleichen.

## Revendications

1. Composition liquide non aqueuse et stable de blanchiment des dents appropriée pour une application sur les dents dans la cavité buccale comprenant un composé peroxide anhydre dispersé dans un polymère liquide hydrophile anhydre oralement acceptable et un agent améliorant l'adhérence, dans laquelle la composition est homogène, le composé peroxyde anhydre est PVP/H₂O₂, le polymère hydrophile anhydre est le polyéthylène glycol et l'agent améliorant l'adhérence est un copolymère de polyvinylpyrrolidone/acétate de vinyle, laquelle composition quand elle est appliquée par voie topique à la surface des dents forme une couche adhérente contenant le composé peroxyde, lequel composé peroxyde est ensuite libéré à partir de la couche pour réaliser un blanchiment des dents.

2. Composition selon la revendication 1, dans laquelle l'agent améliorant l'adhérence est présent dans la composition en une quantité de 0,1 à 4 % en poids.

3. Composition selon la revendication 1, dans laquelle un activateur de peroxide est présent dans la composition.

4. Composition selon la revendication 3, dans laquelle l'activateur de peroxyde est le NaHCO₃, le Na₂CO₃ ou le gluconate de manganèse.

5. Composition selon la revendication 3, dans laquelle l'activateur de peroxyde est présent dans la composition à une concentration de 1 à 10 % en poids.

6. Procédé de blanchiment des dents qui comprend
a) préparer une composition liquide non aqueuse de blanchiment des dents selon la revendication 1 ;
b) peindre la composition en un contact avec les dents à blanchir ;
c) maintenir la composition en contact avec les dents pendant plusieurs minutes par jour ; et ensuite,
d) répéter les étapes b et c pendant plusieurs jours afin de blanchir les dents.
